# EUROPEAN PATENT APPLICATION

(11) **EP 4 336 182 A2**
(43) Date of publication of application: **13.03.2024**
(21) Application number: 24152858.7
(22) Date of filing: 17.04.2020
(51) Int. Cl.: G01N 33/53

(54) **METHODS OF SURFACE MODIFICATION OF SILICONES FOR SPECIFIC TARGET AND HIGH EFFICIENCY BINDING**

(30) Priority: 11.03.2019 US 201962816892 P
(62) Divisional of application: 20770980.9
(71) Applicant: Redbud Labs, Inc., Research Triangle Park, North Carolina 27709 (US)
(72) Inventor: FISHER, Jay Kenneth, 27709 Research Triangle Park (US); KREMER, Katelyn Rose, 27709 Research Triangle Park (US)
(74) Representative: Murgitroyd & Company

(57) **Abstract**

Methods of surface modification of silicones for specific target and high efficiency binding are A surface for target-specific analyst capture is provided, along with a microfluidic device comprising same and a method for functionalizing a surface for target-specific analyst capture. The functionalized surface comprises a binding system comprising a blocking agent and a binding agent.

## Description

### TECHNICAL FIELD OF THE INVENTION

The presently disclosed subject matter relates generally to the processing of biological materials and more particularly to methods of surface modification of silicones for specific target and high efficiency binding; namely, methods of surface modification for high efficiency capture of a target analyte.

### BACKGROUND

Microfluidic devices can include one or more active surfaces, which can be, for example, surface-attached microposts in a reaction chamber that are used for capturing target analytes in a biological fluid. Exemplary microfluidic devices include those described in U.S. Patent Nos. 9,238,869 and 9,612,185, both entitled "Methods and Systems for Using Actuated Surface-Attached Posts for Assessing Biofluid Rheology," which are directed to methods, systems, and computer readable media for using actuated surface-attached posts for assessing biofluid rheology. According to one aspect, a method for testing properties of a biofluid specimen includes placing the specimen onto a micropost array having a plurality of microposts extending outwards from a substrate, wherein each micropost includes a proximal end attached to the substrate and a distal end opposite the proximal end, and generating an actuation force in proximity to the micropost array to actuate the microposts, thereby compelling at least some of the microposts to exhibit motion. The method further includes measuring the motion of at least one of the microposts in response to the actuation force and determining a property of the specimen based on the measured motion of the at least one micropost.

Although microfluidic devices such as those described above have been known, there can still be considerable cost and complexity associated with providing target-specific binding properties to one or more active surfaces (e.g., surface-attached microposts) within the reaction chamber of a microfluidic device. Therefore, new approaches are needed to simplify the process of providing target-specific binding properties to in a microfluidic device used for capturing target analytes in a biological fluid.

### SUMMARY

To address the foregoing problems, in whole or in part, and/or other problems that may have been observed by persons skilled in the art, the present disclosure provides compositions and methods as described by way of example as set forth below.

In one aspect, , a method is provided for functionalizing surface attached microposts to produce a micropost surface for target-specific analyte capture, the method comprising the steps of:
a. providing a micropost processing platform, wherein the micropost processing platform comprises microposts that comprise a silicone-based material; and
b. incorporating one or more functionalizing agents into the silicone-based material of the microposts; whereby the micropost surface for target-specific analyte capture is produced. In some aspects, step 1(b) comprises providing a binding system for binding of a target-specific capture agent, wherein the binding system comprises a generic binding agent and a linking agent. In other aspects, the generic binding agent is biotin and the linking agent is an avidin, and wherein the target-specific capture agent is a biotin-conjugate. In some aspects the avidin is avidin, streptavidin, or neutravidin. In other aspects, the biotin-conjugate is a biotinylated antibody (Ab)). In other aspects, step 1(b) comprises partially or fully functionalizing the surface-attached microposts.

In other aspects, partially or fully functionalizing the surface-attached microposts comprises anchoring one or more functionalizing agents into the silicone-based material of the microposts. In other aspects, anchoring of the one or more functionalizing agents into the silicone-based material of the microposts comprises grafting, bonding, and/or physio-absorption. In other aspects, partially or fully functionalizing the surface-attached microposts comprises providing the one or more functionalizing agents such that the one or more functionalizing agents sit atop the surface of the microposts. In other aspects, the silicone-based material of the microposts comprises PDMS. In other aspects, the silicone-based material of the microposts comprises one or more amine functional groups, wherein the amine functional group provides an anchor point for subsequent addition of the one or more functionalizing agents. In other aspects, the one or more functionalizing agents comprise a binding agent and/or a blocking agent. In other aspects, a generic binding agent is added into the silicone-based material of the microposts. In other aspects, the generic binding agent comprises a biotin-containing agent. In other aspects, the binding agent is a modified lipid that comprises a biotin binding group. In other aspects, the binding agent is 1,2-dioleoyl-sn-glycero-3-phosphoethanolamine-N-(biotinyl) (Bio Dope). In other aspects, the silicone-based material comprises a polymer matrix, and wherein a swelling agent is used to expand the polymer matrix. In other aspects, the swelling agent is removed, whereby the functionalizing agent becomes entangled and anchored in the polymer matrix.

In other aspects, the method further comprises the steps of:
c. reacting functional groups on the surface-attached microposts with a silanizing reagent to form amine-containing groups, whereby amine modified microposts are produced; and
d. causing a crosslinking chemical reaction to add the one or more functionalizing agents to the surface of the amine modified microposts. In other aspects, the silanizing agent comprises 3-aminopropyltriethoxysilane (APTES) and/or 3-glycidyloxypropyltrimethoxysilane (GOPTS). In other aspects, step (d) comprises the use of N-hydroxysuccinimide ester (NHS) and/or 1-Ethyl-3-(3-Dimethylaminopropyl)carbodiimide (EDAC). In other aspects, step (d) comprises the use of click chemistry to conjugate the functionalizing agent to the surface of the microposts. In other aspects, step (d) comprises the use of a silanizing agent that comprises a functionalizing agent. In other aspects, the silanizing agent that comprises a functionalizing agent comprises silane polyethylene glycol (silane PEG). In other aspects, the functionalizing agent comprises a binding group, and further wherein the binding group is a biotin, whereby the functionalizing agent comprises silane polyethylene glycol biotin (silane PEG biotin). In other aspects, step (d) comprises the use of a plasma activation process to generate reactive groups that can be used to anchor the functionalizing agent to the surface of the microposts. In other aspects, the reactive groups comprise hydroxyl groups. In other aspects, step (d) comprises passivating the surface of the microposts, wherein passivating the surface of the microposts comprises reacting unbound amine groups with a blocking agent. In other aspects, step (d) comprises passivating the surface of the microposts, wherein passivating the surface of the microposts comprises incorporating a blocking agent into the silicone-based material that is used to fabricate the microposts.

In other aspects, the blocking agent is selected from the group consisting of: polyethylene glycol (PEG); polyethylene oxide (PEO); a lipid; bovine serum albumin (BSA); polypropylene; and polytetrafluoroethylene (PTFE). In other aspects, a first functionalization reaction is performed to add a generic binding agent on the surface of the modified microposts. In other aspects, a solution of a first functionalization reagent is flowed into the reaction chamber via fluid ports or directly into the gap. In other aspects, the first functionalization reagent is a pegylated amine-reactive biotin reagent (N-hydroxysuccinimide ester (NHS))-PEG-biotin. In other aspects, the PEG spacer arm is configured to act as a "bottlebrush" polymer restricting access to the surfaces of the microposts. whereby non-specific binding to the surfaces of the microposts is reduced. In other aspects, the first functionalization reaction is performed using the first functionalization reagent and reaction conditions sufficient to provide a density of PEG-biotin binding sites on the surface of the microposts sufficient for subsequent high efficiency capture of a target analyte. In other aspects, the first functionalization reagent is a pegylated amine-reactive biotin reagent, and wherein the reagent reacts with amine groups on surfaces of the modified microposts to form stable amide bonds. In other aspects, the chain length of the PEG spacer is selected to provide sufficient mobility to the terminally bound biotin group. In other aspects, the PEG spacer arm is configured to have a molecular weight of between 500 and 5000. In other aspects, the chain length of the blocking PEG molecule is selected to be sufficiently shorter than the chain length of the PEG-biotin binding agent to allow for the mobility of the biotin binding group at the end of the PEG arm.

In other aspects, a micropost processing platform is provided comprising microposts that comprise a silicone-based material and configured for performance of any of the methods described herein. In other aspects, the micropost processing platform comprises a microfluidics system comprising:
i. at least one microfluidic device comprising a reaction chamber, wherein the reaction chamber comprises a micropost field, wherein the micropost field comprises surface-attached magnetically responsive microposts; and
ii. at least one magnetic-based actuation mechanism provided in close proximity to the magnetically responsive microposts, wherein the at least one magnetic-based actuation mechanism is configured to generate an actuation force sufficient to compel at least some of the magnetically responsive microposts to exhibit motion.

In other aspects, the reaction chamber comprises at least one contained microfluidic channel, wherein a defined volume of fluid can be flowed into and out of the micropost processing platform via fluid ports for solution-based functionalization reactions. In other aspects, the reaction chamber comprises multiple microfluidic channels. In other aspects, the method further comprises reacting functional groups on the surface of the silicone-based material of the microposts with APTES to form amine-containing groups on amine modified microposts, followed by the use of one or more amine-ester (NHS) chemical reactions to add one or more functional groups to the surface of the amine modified microposts. In other aspects, the APTES-modification reaction is performed prior to assembly of the microposts processing platform to form a reaction chamber, and wherein the one or more NHS chemical reactions to add one or more functional groups to the surface of the amine modified microposts are performed in the reaction chamber of the assembled microposts processing platform.

In other aspects, the micropost processing platform comprises two facing substrates of functionalized surface-attached microposts. In other aspects, the micropost processing platform comprises at least two individual microfluidics devices configured as drop-in ready modules for integration into a fluidics cartridge or system of an end user. In other aspects, the functionalized surface-attached microposts are configured to be provided to the end user at different stages of the functionalization process depending on end-user requirements. In other aspects, the micropost processing platform comprises a pair of opposing substrates of functionalized surface-attached microposts, wherein the pair of opposing substrates are separated by a gap, thereby forming a reaction chamber between the pair of opposing substrates. In other aspects, the micropost processing platform comprises a spacer or gasket provided between the pair of substrates to form the gap. In other aspects, the height of the gap is from about 50 µm to about 1 mm.

In other aspects, the micropost processing platform is configured to allow bulk fluid to be flowed through the reaction chamber by supplying fluid directly to the gap. In other aspects, one end of the reaction chamber is an inlet and the other end of the reaction chamber is an outlet. In other aspects, the micropost processing platform comprises fluid ports provided in one or both substrates. In other aspects, the micropost processing platform comprises two fluid ports provided in one substrate, wherein one fluid port is an inlet and the other inlet port is an outlet.

In other aspects, the micropost processing platform comprises:
i. at least one microfluidic device comprising a reaction chamber, wherein the reaction chamber comprises a micropost field, wherein the micropost field comprises surface-attached magnetically responsive microposts; and
ii. at least one magnetic-based actuation mechanism provided in close proximity to the magnetically responsive microposts, wherein the at least one magnetic-based actuation mechanism is configured to generate an actuation force sufficient to compel at least some of the magnetically responsive microposts to exhibit motion.

In other aspects, each of the at least one microfluidic devices comprises a bottom substrate and a top substrate separated by a gap, thereby forming the reaction chamber between the bottom substrate and the top substrate. In other aspects, a spacer or gasket is provided between the bottom substrate and top the substrate to form the gap and define the reaction chamber. In other aspects, the top substrate comprises an inlet port and an outlet port. In other aspects, the reaction chamber is sized to hold a desired volume of fluid. In other aspects, the gap has a height of from about 50 µm to about 1 mm.

In other aspects, the micropost processing platform comprises a micropost field provided on the inner surface of the bottom substrate. In other aspects, the micropost field is provided on the inner surface of the top substrate. In other aspects, the micropost field is provided on both the inner surface of the bottom substrate and the inner surface of the top substrate. In other aspects, the microposts within the reaction chamber are functionalized with target-specific analyte capture elements. In other aspects, the microposts are functionalized using a surface-functionalization process. In other aspects, surface functionalization of the microposts is performed prior to assembly of the two substrates to form the reaction chamber.

In other aspects, the micropost processing platform comprises opposing microposts, wherein the opposing microposts are configured such that there is no space or gap between the tips of the opposing microposts. In other aspects, the opposing microposts are interdigitated. In other aspects, the interdigitated opposing microposts are configured such that the tips of the opposing microposts are set at different planes such that they overlap. In other aspects, the tips of a first set of the opposing microposts are set at a plane p1 and the tips of an opposing set of microposts to the first set of microposts are set at a plane p2. In other aspects, the interdigitated opposing microposts are configured such that the tips of the opposing microposts are set with substantially no overlap. In other aspects, the tips of the opposing microposts are set at the same plane. In other aspects, the tips of the opposing microposts are separated by a space or gap. In other aspects, the tips of the opposing microposts are configured such that the space or gap between the tips of the opposing microposts provides a low resistance flow path through the center of the reaction chamber through which targets can travel and not encounter a micropost.

Other compositions, methods, features, and advantages of the invention will be or will become apparent to one with skill in the art upon examination of the following figures and detailed description. It is intended that all such additional compositions, methods, features, and advantages be included within this description, be within the scope of the invention, and be protected by the accompanying claims.

### BRIEF DESCRIPTION OF THE DRAWINGS

The features and advantages of the present invention will be more clearly understood from the following description taken in conjunction with the accompanying drawings, which are not necessarily drawn to scale, and wherein:
FIG. 1A and FIG. 1B illustrate cross-sectional views of an example of a microposts processing platform, wherein the microposts processing platform provides a reaction (or assay) chamber for processing an arrangement of microposts;
FIG. 1C illustrates examples of a Detail A of FIG. 1A that shows more details of the microposts processing platform;
FIG. 2A and FIG. 2B illustrate cross-sectional views of another example of a microposts processing platform, wherein the microposts processing platform provides a reaction (or assay) chamber for processing an arrangement of microposts;
FIG. 3A and FIG. 3B illustrate a perspective view and a cross-sectional view, respectively, of an example of a substrate with surface-attached microposts formed thereon;
FIG. 4A and FIG. 4B illustrate side views of an example of microposts of themicropostbased reaction chamber of the microposts processing platform;
FIG. 5A and FIG. 5B illustrate side views of a micropost and show examples of the actuation motion thereof;
FIG. 6 illustrates a flow diagram of an example of the presently disclosed method of surface modification of silicones for specific target binding and high efficiency binding;
FIG. 7A and FIG. 7B show pictorially some of the steps of the method of FIG. 6;
FIG. 8 illustrates a flow diagram of another example of the presently disclosed method of surface modification of silicones for specific target binding and high efficiency binding;
FIG. 9 illustrates a flow diagram of yet another example of the presently disclosed method of surface modification of silicones for specific target binding and high efficiency binding;
FIG. 10A and FIG. 10B are photos of analyte capture experiments performed using beads suspended in a simple buffer matrix and bacteria (i.e., *E. coli*) spiked in whole blood, respectively; and
FIG. 11, FIG. 12, and FIG. 13 show views of various examples of processed microposts platforms that include functionalized microposts according to the presently disclosed methods of surface modification of silicones for specific target binding and high efficiency binding.

### DETAILED DESCRIPTION

The presently disclosed subject matter now will be described more fully hereinafter with reference to the accompanying Drawings, in which some, but not all embodiments of the presently disclosed subject matter are shown. Like numbers refer to like elements throughout. The presently disclosed subject matter may be embodied in many different forms and should not be construed as limited to the embodiments set forth herein; rather, these embodiments are provided so that this disclosure will satisfy applicable legal requirements. Indeed, many modifications and other embodiments of the presently disclosed subject matter set forth herein will come to mind to one skilled in the art to which the presently disclosed subject matter pertains having the benefit of the teachings presented in the foregoing descriptions and the associated Drawings. Therefore, it is to be understood that the presently disclosed subject matter is not to be limited to the specific embodiments disclosed and that modifications and other embodiments are intended to be included within the scope of the appended claims.

### General Definitions

As used herein "active surface" means any surface or area that can be used for processing samples including, but not limited to, biological materials, fluids, environmental samples (e.g., water samples, air samples, soil samples, solid and liquid wastes, and animal and vegetable tissues), and industrial samples (e.g., food, reagents, and the like). The active surface can be inside a reaction or assay chamber. For example, the active surface can be any surface thathas properties designed to manipulate the fluid inside the chamber. Manipulation can include, for example, generating fluid flow, altering the flow profile of an externally driven fluid, fractionatingthe sample into constituent parts, establishing or eliminating concentration gradients within the chamber, and the like. Surface properties that might have this effect can include, for example, post technology - whether static or actuated (i.e., activated). The surface properties may also include microscale texture or topography in the surface, physical perturbation of the surface by vibration or deformation; electrical, electronic, electromagnetic, and/or magnetic system on or in the surface; optically active (e.g., lenses) surfaces, such as embedded LEDs or materials that interact with external light sources; and the like.

As used herein, the terms "surface-attached post" or "surface-attached micropost" or "surface-attached structure" are used interchangeably. Generally, a surface-attached structure has two opposing ends: a fixed end and a free end. The fixed end may be attached to a substrate by any suitable means, depending on the fabrication technique and materials employed. The fixed end may be "attached" by being integrally formed with or adjoined to the substrate, such as by a microfabrication process. Alternatively, the fixed end may be "attached" via a bonding, adhesion, fusion, or welding process. The surface-attached structure has a length defined from the fixed end to the free end, and a cross-section lying in a plane orthogonal to the length. For example, using the Cartesian coordinate system as a frame of reference, and associating the length of the surface-attached structure with the z-axis (which may be a curved axis), the cross-section of the surface-attached structure lies in the x-y plane.

Generally, the cross-section of the surface-attached structure may have any shape, such as rounded (e.g., circular, elliptical, etc.), polygonal (or prismatic, rectilinear, etc.), polygonal with rounded features (e.g., rectilinear with rounded corners), or irregular. The size of the cross-section of the surface-attached structure in the x-y plane may be defined by the "characteristic dimension" of the cross-section, which is shape-dependent. As examples, the characteristic dimension may be diameter in the case of a circular cross-section, major axis in the case of an elliptical cross-section, or maximum length or width in the case of a polygonal cross-section. The characteristic dimension of an irregularly shaped cross-section may be taken to be the dimension characteristic of a regularly shaped cross-section that the irregularly shaped cross-section most closely approximates (e.g., diameter of a circle, major axis of an ellipse, length or width of a polygon, etc.).

A surface-attached structure as described herein is non-movable (static, rigid, etc.) or movable (flexible, deflectable, bendable, etc.) relative to its fixed end or point of attachment to the substrate. To facilitate the movability of movable surface-attached structures, the surface-attached structure may include a flexible body composed of an elastomeric (flexible) material, and may have an elongated geometry in the sense that the dominant dimension of the surface-attached structure is its length-that is, the length is substantially greater than the characteristic dimension. Examples of the composition of the flexible body include, but are not limited to, elastomeric materials such as hydrogel and other active surface materials (for example, polydimethylsiloxane (PDMS)).

The movable surface-attached structure is configured such that the movement of the surface-attached structure relative to its fixed end may be actuated or induced in a non-contacting manner, specifically by an applied magnetic or electric field of a desired strength, field line orientation, and frequency (which may be zero in the case of a magnetostatic or electrostatic field). To render the surface-attached structure movable by an applied magnetic or electric field, the surface-attached structure may include an appropriate metallic component disposed on or in the flexible body of the surface-attached structure. To render the surface-attached structure responsive to a magnetic field, the metallic component may be a ferromagnetic material such as, for example, iron, nickel, cobalt, or magnetic alloys thereof, one non-limiting example being "alnico" (an iron alloy containing aluminum, nickel, and cobalt). To render the surface-attached structure responsive to an electric field, the metallic component may be a metal exhibiting good electrical conductivity such as, for example, copper, aluminum, gold, and silver, and well as various other metals and metal alloys. Depending on the fabrication technique utilized, the metallic component may be formed as a layer (or coating, film, etc.) on the outside surface of the flexible body at a selected region of the flexible body along its length. The layer may be a continuous layer or a densely grouped arrangement of particles. Alternatively, the metallic component may be formed as an arrangement of particles embedded in the flexible body at a selected region thereof.

As used herein, the term "actuation force" refers to the force applied to the microposts. For example, the actuation force may include a magnetic, thermal, sonic, or electric force. Notably, the actuation force may be applied as a function of frequency or amplitude, or as an impulse force (i.e., a step function). Similarly, other actuation forces may be used without departing from the scope of the present subject matter, such as fluid flow across the micropost array (e.g., flexible microposts that are used as flow sensors via monitoring their tilt angle with an optical system).

Accordingly, the application of an actuation force actuates the movable surface-attached microposts into movement. For example, the actuation occurs by contacting cell processing chamber with the control instrument comprising elements that provide an actuation force, such as a magnetic or electric field. Accordingly, the control instrument includes, for example, any mechanisms for actuating the microposts (e.g., magnetic system), any mechanisms for counting the cells (e.g., imaging system), the pneumatics for pumping the fluids (e.g., pumps, fluid ports, valves), and a controller (e.g., microprocessor).

As used herein, a "flow cell" is any chamber comprising a solid surface across which one or more liquids can be flowed, wherein the chamber has at least one inlet and at least one outlet.

The term "micropost array" is herein used to describe an array of small posts, extending outwards from a substrate, that typically range from 1 to 100 micrometers in height. In one embodiment, microposts of a micropost array may be vertically-aligned. Notably, each micropost includes a proximal end that is attached to the substrate base and a distal end or tip that is opposite the proximal end. Microposts may be arranged in arrays such as, for example, the microposts described in U.S. Patent 9,238,869, entitled "Methods and systems for using actuated surface-attached posts for assessing biofluid rheology," issued on January 19, 2016; the entire disclosure of which is incorporated herein by reference. U.S. Patent No. 9,238,869 describes methods, systems, and computer readable media for using actuated surface-attached posts for assessing biofluid rheology. One method described in U.S. Patent No. 9,238,869 is directed to testing properties of a biofluid specimen that includes placing the specimen onto a micropost array having a plurality of microposts extending outwards from a substrate, wherein each micropost includes a proximal end attached to the substrate and a distal end opposite the proximal end, and generating an actuation force in proximity to the micropost array to actuate the microposts, thereby compelling at least some of the microposts to exhibit motion. This method further includes measuring the motion of at least one of the microposts in response to the actuation force and determining a property of the specimen based on the measured motion of the at least one micropost.

U.S. Patent No. 9,238,869 also states that the microposts and micropost substrate of the micropost array can be formed of polydimethylsiloxane (PDMS). Further, microposts may include a flexible body and a metallic component disposed on or in the body, wherein application of a magnetic or electric field actuates the microposts into movement relative to the surface to which they are attached (e.g., wherein the actuation force generated by the actuation mechanism is a magnetic and/or electrical actuation force).

"Magnetically responsive" means responsive to a magnetic field. "Magnetically responsive microposts" include or are composed of magnetically responsive materials. Examples of magnetically responsive materials include, but are not limited to, paramagnetic materials, ferromagnetic materials, ferrimagnetic materials, and metamagnetic materials. Examples of suitable paramagnetic materials include iron, nickel, and cobalt, as well as metal oxides, such as, but not limited to, ferroferric oxide (Fe3O4), barium hexaferrite (BaFe12O19), cobalt(II) oxide (CoO), nickel(II) oxide (NiO), manganese(III) oxide (Mn2O3), chromium(III) oxide (Cr2O3), and cobalt manganese phosphide (CoMnP).

Following long-standing patent law convention, the terms "a," "an," and "the" refer to "one or more" when used in this application, including the claims. Thus, for example, reference to "a subject" includes a plurality of subjects, unless the context clearly is to the contrary (e.g., a plurality of subjects), and so forth.

Throughout this specification and the claims, the terms "comprise," "comprises," and "comprising" are used in a non-exclusive sense, except where the context requires otherwise. Likewise, the term "include" and its grammatical variants are intended to be non-limiting, such that recitation of items in a list is not to the exclusion of other like items that can be substituted or added to the listed items.

For the purposes of this specification and appended claims, unless otherwise indicated, all numbers expressing amounts, sizes, dimensions, proportions, shapes, formulations, parameters, percentages, quantities, characteristics, and other numerical values used in the specification and claims, are to be understood as being modified in all instances by the term "about" even though the term "about" may not expressly appear with the value, amount or range. Accordingly, unless indicated to the contrary, the numerical parameters set forth in the following specification and attached claims are not and need not be exact, but may be approximate and/or larger or smaller as desired, reflecting tolerances, conversion factors, rounding off, measurement error and the like, and other factors known to those of skill in the art depending on the desired properties sought to be obtained by the presently disclosed subject matter. For example, the term "about," when referring to a value can be meant to encompass variations of, in some embodiments, ± 100% in some embodiments ± 50%, in some embodiments ± 20%, in some embodiments ± 10%, in some embodiments ± 5%, in some embodiments ±1%, in some embodiments ± 0.5%, and in some embodiments ± 0.1% from the specified amount, as such variations are appropriate to perform the disclosed methods or employ the disclosed compositions.

Further, the term "about" when used in connection with one or more numbers or numerical ranges, should be understood to refer to all such numbers, including all numbers in a range and modifies that range by extending the boundaries above and below the numerical values set forth. The recitation of numerical ranges by endpoints includes all numbers, e.g., whole integers, including fractions thereof, subsumed within that range (for example, the recitation of 1 to 5 includes 1, 2, 3, 4, and 5, as well as fractions thereof, e.g., 1.5, 2.25, 3.75, 4.1, and the like) and any range within that range.

### Methods of Surface Modification of Silicones for Specific Target and High Efficiency Binding

In some embodiments, the presently disclosed subject matter provides methods of surface modification of silicones for specific target and high efficiency binding. Namely, a surface-functionalization process uses a microposts processing platform to functionalize microposts for high efficiency capture of a target analyte. High efficiency binding can mean, for example, rapid binding and/or successful binding to the majority of the binding sites. For example, a microposts processing platform is provided for facilitating a micropost surface-functionalization process. The microposts processing platform is, for example, based on a microfluidic flow cell structure formed by two opposing substrates that form a reaction (or assay) chamber and wherein the surface of one or both of the substrates includes an arrangement (e.g., a field or array) of the surface-attached microposts to be functionalized. The surface-attached microposts are formed of a silicone-based material (e.g., polydimethylsiloxane (PDMS)) and functionalized with a binding agent for target-specific analyte capture.

In some embodiments, the opposing microposts on the opposing substrates are arranged in "interdigitated" fashion with an overlap. Namely, the tips of the opposing microposts are arranged in "interdigitated" fashion with substantially no space or gap therebetween. In this embodiment, there is substantially no low resistance flow path between the opposing microposts. However, in other embodiments, the tips of the opposing microposts on the opposing substrates are arranged with a space or gap therebetween. In this embodiment, there is a low resistance flow path between the opposing microposts.

In some embodiments, the methods of functionalizing the surface-attached microposts using the microposts processing platform include one or more modification/functionalization steps, wherein the incorporation of one or more functional (e.g., binding or blocking) agents are used to provide a micropost surface for target-specific analyte capture.

In some embodiments, the micropost surface-functionalization process includes providing a binding system, wherein the binding system includes a generic binding agent and a linking agent for binding of a target-specific capture agent. In one example, the generic binding agent is biotin and the linking agent is avidin (e.g., avidin, streptavidin, neutravidin) and the target-specific capture agent is a biotin-conjugate (e.g., a biotinylated antibody (Ab)).

In some embodiments, the surface-attached microposts can be partially or fully functionalized by integration (anchoring) of a functional group into the silicone-based material (e.g., PDMS) used to fabricate the microposts. For example, the silicone-based material (e.g., PDMS) used to fabricate the microposts can include a functional group as part of the polymer chain. In one example, the silicone-based material (e.g., PDMS) used to fabricate the microposts can include amine functional groups, wherein the amine functional group provides an anchor point for subsequent addition of a functionalizing agent (e.g., a binding agent and/or blocking agent). In another example, an anchoring molecule that includes a generic binding agent (e.g., a biotin-containing agent) can be added (doped) into the silicone-based material (e.g., PDMS) that is used to fabricate the microposts. In one example, the anchoring molecule is a modified lipid that includes a biotin binding group (e.g., 1-oleoyl-2-[12-biotinyl(aminododecanoyl)]-sn-glycero-3-phosphocholine, available from Avanti Lipids), wherein the biotin-modified lipid is incorporated into the micropost PDMS matrix. In yet another example, a swelling agent, such as dichloromethane, is used to expand the polymer matrix of the silicone-based material (e.g., PDMS) creating space between the polymer chains, wherein a functionalizing agent (e.g., a binding agent and/or blocking agent) is entangled and anchored in the polymer matrix upon removal of the swelling agent.

In some embodiments, the arrangement of surface-attached microposts can be functionalized using a surface-functionalization process. Namely, a functionalizing agent (e.g., a binding and/or blocking agent) is attached (anchored) to (e.g., grafted, bonded, physio-absorbed etc.) or sits atop the surface of a micropost.

In some embodiments, a micropost surface-functionalization process includes reacting functional groups (e.g., silanol and radical groups) on the surface of native microposts (e.g., PDMS microposts) with a silanizing reagent to form amine-containing groups and then using one or more crosslinking (conjugating) chemistries to add (anchor) one or more functionalizing agents (e.g., binding and/or blocking agent) to the surface of the amine modified microposts. Non-limiting examples of silanizing agents include: 3-aminopropyltriethoxysilane (APTES), (3-Aminopropyl)trimethoxysilane (APTMS), and 3-glycidyloxypropyltrimethoxysilane (GOPTS). Non-limiting examples of crosslinker (conjugating) chemistries include: N-hydroxysuccinimide ester (NHS), and 1-Ethyl-3-(3-Dimethylaminopropyl)carbodiimide (EDAC).

In some embodiments, a micropost surface-functionalization process can be performed using "click" chemistry to conjugate a functionalizing agent (e.g., binding and/or blocking agent) to the surface of the microposts. Non-limiting examples of click chemistries for conjugation include the reaction of an azide with an alkyne group.

In some embodiments, a micropost surface-functionalization process can be performed using a silanizing agent that includes a functionalizing agent (e.g., binding and/or blocking agent). In one example, silane polyethylene glycol (silane PEG) that includes a binding group such as biotin (i.e., silane PEG biotin) can be used to anchor a binding group on the surface of the microposts.

In some embodiments, a micropost surface-functionalization process can be performed using a plasma activation process to generate reactive groups (e.g., hydroxyl groups) that can be used to anchor a functionalizing agent (e.g., binding and/or blocking agent) on the surface of the microposts.

In some embodiments, the micropost surface-functionalization process includes passivating the surface of the microposts. In one example, the micropost surface-functionalization process includes reacting unbound amine groups with a blocking (passivating) agent. In another example, click chemistry can be used to conjugate a blocking agent to the surface of the microposts. In another example, passivating the surface of the microposts can be performed by incorporating (doping) a blocking (passivating) agent into the silicone-based material (e.g., PDMS) that is used to fabricate the microposts. Non-limiting examples of blocking (passivation) agents include: polyethylene glycol (PEG; or polyethylene oxide (PEO)), lipids, bovine serum albumin (BSA), polypropylene, and polytetrafluoroethylene (PTFE).

In some embodiments, the micropost surface-functionalization process can include one or more steps that are performed prior to assembly of the reaction (or assay) chamber of the microposts processing platform and one or more solution-based steps within the reaction (or assay) chamber of the assembled microposts processing platform. The reaction chamber provides a contained microfluidic channel, wherein a defined volume of fluid can be flowed into and out of the microposts processing platform via fluid ports for solution-based functionalization reactions. The solution-based functionalization reactions can be performed using a single microfluidic channel or scaled to use multiple microfluidic channels. Because the solution-based functionalization reactions are performed in the defined space of a microfluidic channel (chamber), only microposts within the reaction chamber are exposed to the functionalization reagents, thereby holding reagent volumes and costs to a minimum.

In some embodiments, a micropost surface-functionalization process includes reacting functional groups (e.g., silanol and radical groups) on the surface of the native microposts (e.g., PDMS microposts) with APTES to form amine-containing groups and then using one or more amine-ester (NHS) chemical reactions to add (anchor) one or more functional groups (e.g., binding groups or blocking groups) to the surface of the amine modified microposts. In one example, the APTES-modification reaction is performed prior to assembly of the microposts processing platform to form a reaction (or assay) chamber and the one or more amine-ester (NHS) chemical reactions to add one or more functional groups (e.g., binding groups or blocking groups) to the surface of the amine modified microposts are performed in the reaction (or assay) chamber of the assembled microposts processing platform.

In some embodiments, the presently disclosed microposts processing platform with functionalized surface-attached microposts therein can be provided to the end user (1) in its entirety; namely, as a whole microposts processing platform including the two facing substrates of functionalized surface-attached microposts, or (2) as individual microfluidics devices (e.g., diced from the larger microposts processing platform) that can be a drop-in ready module for easily integrating into any fluidics cartridges or systems of an end user. Additionally, the microposts processing platform with the functionalized surface-attached microposts or the individual microfluidics devices with the functionalized surface-attached microposts can be provided to the end user at different stages of the functionalization process depending on the end-user requirements.

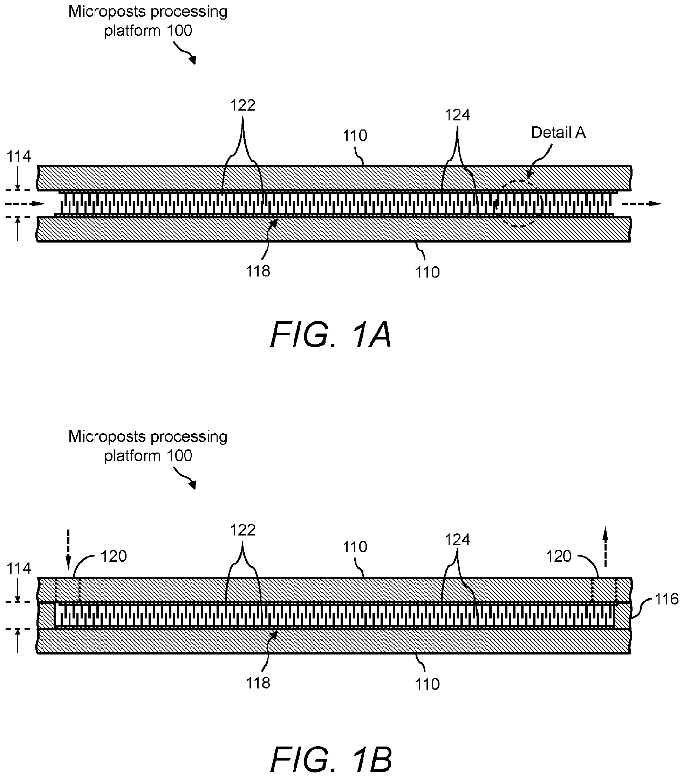

Referring now to FIG. 1A and FIG. 1B is cross-sectional views of an example of a microposts processing platform 100 for processing the presently disclosed functionalized surface-attached microposts for target analyte capture. Microposts processing platform 100 has a reaction (or assay) chamber for processing a plurality of microposts. For example, microposts processing platform 100 includes a pair of opposing substrates 110 separated by a gap 114, thereby forming the reaction (or assay) chamber 118 between the pair of substrates 110. A spacer or gasket 116 (see FIG. 1B) may be provided between the pair of substrates 110 to form gap 114 and define the area of reaction (or assay) chamber 118. Accordingly, a reaction (or assay) chamber 118 is formed in the space between the pair of substrates 110. Reaction (or assay) chamber 118 of microposts processing platform 100 can be sized to hold any volume of fluid. The height of gap 114 of reaction (or assay) chamber 118 can be, for example, from about 50 µm to about 1 mm.

In one example and referring now to FIG. 1A, bulk fluid can be flowed through reaction (or assay) chamber 118 by supplying fluid directly to the gap 114 of reaction (or assay) chamber 118. For example, one end (or side) of reaction (or assay) chamber 118 can be the inlet while the other end (or side) can be the outlet. In another example and referring now to FIG. 1B, fluid ports 120 can be provided in one or both substrates 110. For example, two fluid ports 120 are provided in one substrate 110, one at each end (e.g., an inlet and an outlet) for loading fluid into reaction (or assay) chamber 118. In this example, microposts processing platform 100 provides a simple "flow cell" type of chamber. For example, a flow cell can be any chamber comprising a solid surface across which one or more liquids can be flowed, wherein the chamber has at least one inlet and atleast one outlet.

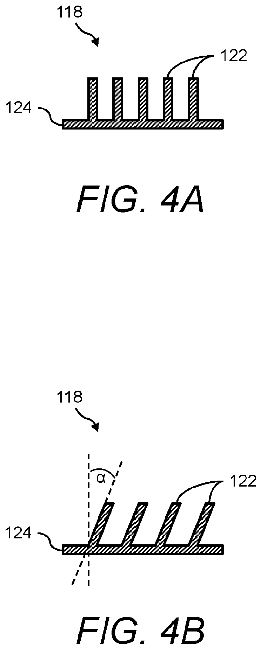

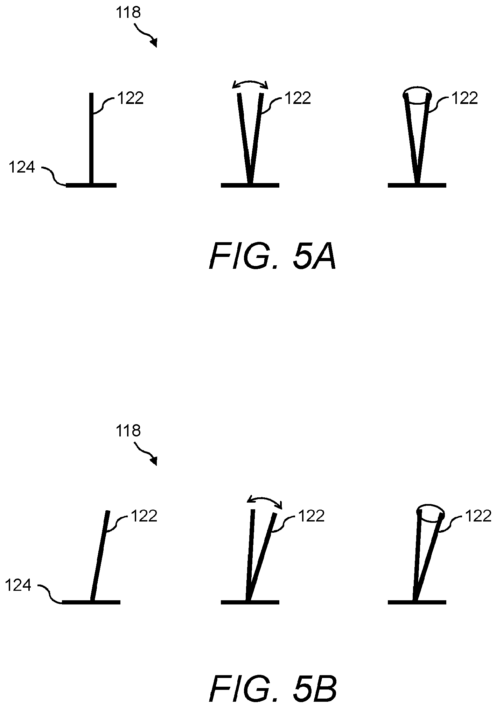

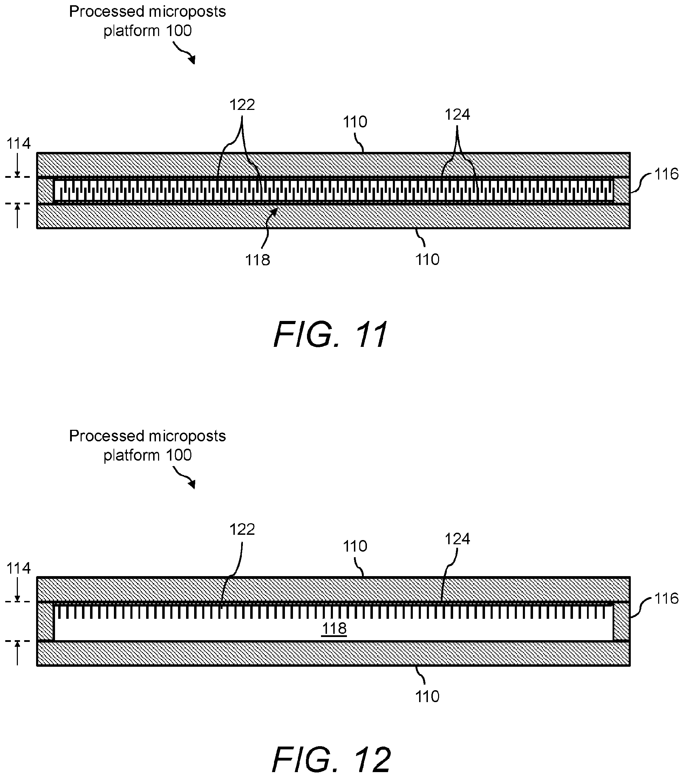

An arrangement (e.g., a field or array) of surface-attached microposts 122 on a substrate 124 is provided on the inner surface of both of the two substrates 110 and within reaction (or assay) chamber 118. However, in other embodiments, the surface-attached microposts 122 can be provided on one substrate 110 only (see FIG. 12). Microposts 122 can be, for example, magnetically responsive microposts. A magnetic actuation mechanism (not shown) can be arranged in close proximity to reaction (or assay) chamber 118 of microposts processing platform 100, wherein a magnetic field from the actuation mechanism can be used to actuate the magnetically responsive microposts 122. More details of magnetic actuation mechanisms are described with reference to the U.S. Patent App. No. 62/654,048, entitled "Magnetic-Based Actuation Mechanisms for and Methods of Actuating Magnetically Responsive Microposts in a Reaction Chamber." More details of microposts 122 are shown and described hereinbelow with reference to FIG. 4A, FIG. 4B, FIG. 5A, and FIG. 5B.

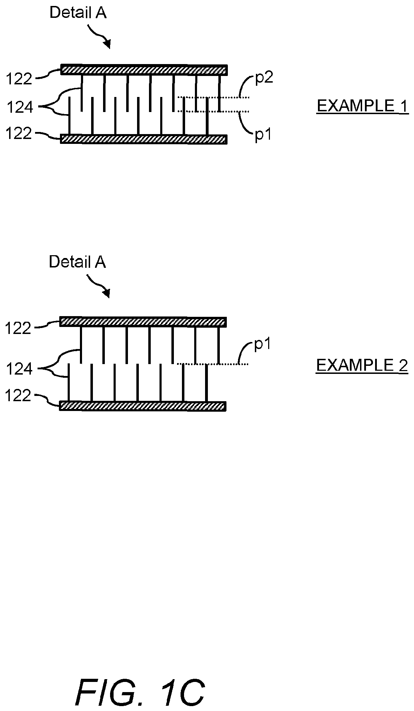

In the example shown in FIG. 1A and FIG. 1B there is no space or gap between the tips of the opposing microposts 122. One way to achieve this is to arrange the microposts 122 of the first substrate 110 in "interdigitated" fashion with respect to the microposts 122 of the second substrate 110. A Detail A of FIG. 1A indicates a portion of microposts processing platform 100. Referring now to FIG. 1C is two examples of Detail A of FIG. 1A; namely, a magnified portion of the microposts processing platform 100 and showing more details thereof. In EXAMPLE 1, the opposing microposts 122 are arranged in "interdigitated" fashion with an overlap. Namely, the tips of the opposing microposts 122 are set at different planes such that they overlap. For example, the tips of one set of microposts 122 are set at a plane p1, while the tips of the opposing set of microposts 122 are set at a plane p2. In EXAMPLE 2, the opposing microposts 122 are likewise arranged in "interdigitated" fashion, but with substantially no overlap and yet with substantially no gap therebetween. Namely, the tips of the opposing microposts 122 are set at substantially the same plane. For example, the tips of one set of microposts 122 are set at the plane p1, while the tips of the opposing set of microposts 122 are set at the same plane p1.

In both EXAMPLE 1 and EXAMPLE 2 there is substantially no space or gap between the tips of the opposing microposts 122. These configurations ensure that there is no low resistance flow path within reaction (or assay) chamber 118 and ensure efficient processing operation. Wherein, for example, beads/capture targets may flow during processing. Namely, this configuration ensures that substantially all fluid and its constituents flow through (and not around) the field of microposts 122.

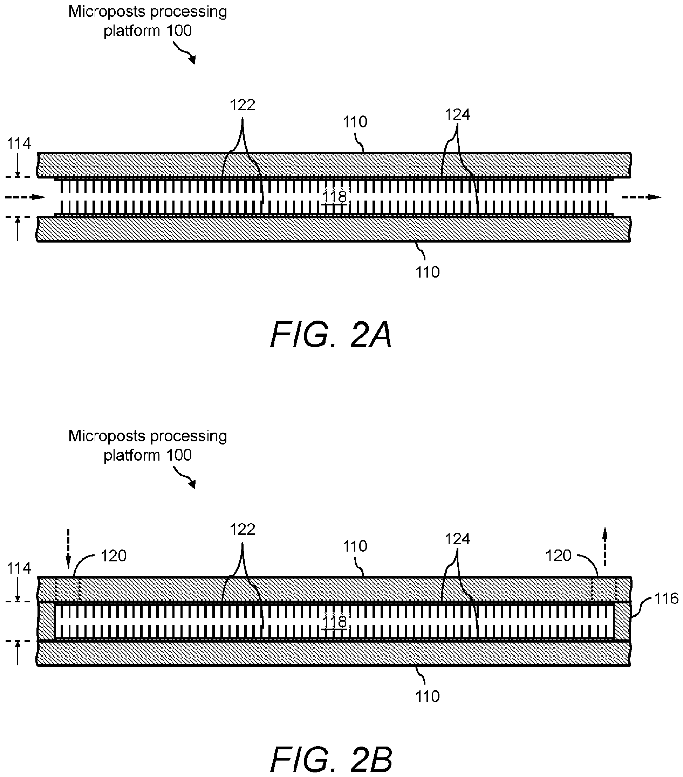

In another configuration, a space or gap may be present between the tips of the opposing microposts 122. Referring now to FIG. 2A and FIG. 2B is cross-sectional views of another example of a microposts processing platform 100, wherein a space or gap is present between the tips of the opposing microposts 122. Wherein this configuration may be less efficient than the configurations shown in FIG. 1A, FIG. 1B, and FIG. 1C, this configuration may be useful. In this example, the space or gap between the tips of the opposing microposts 122 provides a low resistance flow path through the center of reaction (or assay) chamber 118 (where fluid velocity will be the highest) through which targets (e.g., beads/capture targets) can travel and not encounter a micropost 122 in x/y.

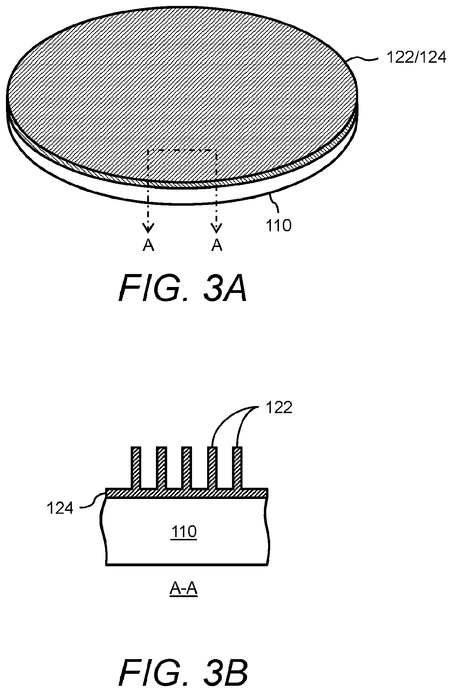

Referring now to FIG. 3A and FIG. 3B is a perspective view and a cross-sectional view, respectively, of an example of substrate 110 with surface-attached microposts 122 formed thereon. Namely, FIG. 3B is a cross-sectional view taken along line A-A of FIG. 3A. Substrate 110 can be, for example, a 6-inch or 12-inch diameter wafer that has a substantially continuous field or array of free-standing microposts 122 across its area. Substrate 110 can be a rigid or semi-rigid substrate formed, for example, of glass, plastic, silicon, or silicone. In one example, each of the substrates 110 is a plastic substrate, such as a substrate formed of the semi-rigid Melinex^{®} brand polyester film available from DuPont Teijin Films (Chester, VA). The thickness of the Melinex^{®} substrate 110 can be from about 100 µm to about 300 µm in one example, or is about 250 µm in another example.

Referring now to FIG. 4A and FIG. 4B is side views of an example of a portion of reaction (or assay) chamber 118, wherein microposts 122 can be arranged in a micropost field or array. The term "micropost field" or "micropost array" is herein used to describe a field or an array of small posts, extending outwards from a substrate, that typically range from 1 to 100 micrometers in height. In one embodiment, microposts of a micropost field or array may be vertically-aligned. Notably, each micropost includes a proximal end that is attached to the substrate base and a distal end or tip that is opposite the proximal end. Accordingly, an arrangement of microposts 122 are provided on a substrate124.

Microposts 122 and substrate 124 can be formed, for example, of polydimethylsiloxane (PDMS). The length, diameter, geometry, orientation, and pitch of microposts 122 in the field or array can vary. For example, the length of microposts 122 can vary from about 1 µm to about 100 µm. The diameter of microposts 122 can vary from about 0.1 µm to about 10 µm. Further, the cross-sectional shape of microposts 122 can vary. For example, the cross-sectional shape of microposts 122 can be circular, ovular, square, rectangular, triangular, and so on. The orientation of microposts 122 can vary. For example, FIG. 4A shows microposts 122 oriented substantially normal to the plane of substrate 124, while FIG. 4B shows microposts 122 oriented at an angle α with respect to normal of the plane of substrate 124. In a neutral position with no actuation force applied, the angle α may be, for example, from about 0 degrees to about 45 degrees. Additionally, the pitch of microposts 122 within a micropost field or array can vary, for example, from about 0 µm to about 50 µm. Further, the relative positions of microposts 122 within the micropost field or array can vary.

FIG. 5A and FIG. 5B show side views of a micropost 122 and show examples of the actuation motion thereof. Namely, FIG. 5A shows an example of a micropost 122 oriented substantially normal to the plane of substrate 124. FIG. 5A shows that the distal end of the micropost 122 can move (1) with side-to-side 2D motion only with respect to the fixed proximal end or (2) with circular motion with respect to the fixed proximal end, which is a cone-shaped motion. By contrast, FIG. 5B shows an example of a micropost 122 oriented at an angle with respect to the plane of substrate 124. FIG. 5B shows that the distal end of the micropost 122 can move (1) with tilted side-to-side 2D motion only with respect to the fixed proximal end or (2) with tilted circular motion with respect to the fixed proximal end, which is a tilted cone-shaped motion. In any microposts processing platform 100, by actuating microposts 122 and causing motion thereof, any fluid in reaction (or assay) chamber 118 is in effect stirred or caused to flow or circulate. Further, the cone-shaped motion of micropost 122 shown in FIG. 5A, as well as the tilted cone-shaped motion of micropost 122 shown in FIG. 5B, can be achieved using a rotating magnetic field. A rotating magnetic field is one example of the "actuation force" of a microposts actuation mechanism.

Referring still to FIG. 1A through FIG. 5B, microposts 122 are based on, for example, the microposts described in the U.S. Patent 9,238,869, entitled "Methods and systems for using actuated surface-attached posts for assessing biofluid rheology," the entire disclosure of which is incorporated herein by reference. In one example, according to the '869 patent, microposts 122 and substrate 124 can be formed of PDMS.

In various embodiments, microposts 122 within reaction (or assay) chamber 118 of microposts processing platform 100 can be functionalized with target-specific analyte capture elements. For example, microposts 122 can be functionalized with analyte capture elements, as described, for example, with reference to U.S. Patent App. No. 62/220,906, entitled "Flow Cells Utilizing Surface-Attached Structures, and Related Systems and Methods;" the entire disclosure of which is incorporated herein by reference. Accordingly, by functionalizing microposts 122 with analyte capture elements, microposts processing platform 100 can be used for affinity-based binding or separation operations.

In some embodiments, microposts 122 can be functionalized using a surface-functionalization process. Namely, the functional group (e.g., binding or blocking group) is attached to (e.g., grafted, bonded, etc.) or sits atop the surface of microposts 122. In one example, surface functionalization of the field or array of microposts 122 in microposts processing platform 100 can be performed prior to assembly of the two substrates 110 to form reaction (or assay) chamber 118 with microposts 122 therein. In another example, the surface-functionalization process of microposts 122 can include one or more steps performed prior to assembly of the two substrates 110 to form reaction (or assay) chamber 118 with microposts 122 therein.

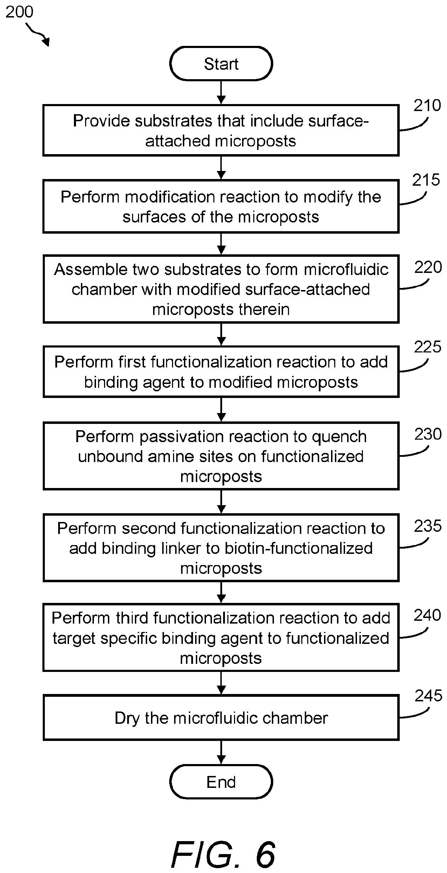

FIG. 6 illustrates a flow diagram of an example of the presently disclosed method 200 of surface modification of silicones for specific target binding and high efficiency binding. Namely, method 200 uses a surface-functionalization process for functionalizing microposts 122 of microposts processing platform 100 for high efficiency capture of a target analyte using a biotinavidin binding system. Method 200 may include, but is not limited to, the following steps.

At a step 210, one or more substrates that include surface-attached microposts are provided. For example and referring now to FIG. 1A through FIG. 2B, one or more substrates 110 are provided wherein at least one substrate 110 includes unprocessed surface-attached microposts 122. In one example, microposts 122 are formed of PDMS.

At a step 215, a modification reaction is performed to modify the surfaces of the PDMS microposts 122. For example, using a vapor deposition process, the one or more substrates 110 with surface-attached microposts 122 thereon are placed in a reaction chamber and exposed to a silanizing agent to convert functional groups (e.g., silanol and radical groups) on the surfaces of PDMS microposts 122 to amine containing groups. In one example, the silanizing agent is 3-aminopropyltriethoxysilane (APTES). The modification reaction is performed such that all or substantially all the micropost 122 surfaces are exposed to the silanizing agent (i.e., APTES) and the PDMS functional groups (e.g., silanol and radical groups) are converted to amine containing groups (i.e., amine-functionalized).

At a step 220, the two substrates are assembled to form a microfluidic chamber with modified surface-attached microposts therein. For example and referring still to FIG. 1A through FIG. 2B, the two substrates 110 and spacer or gasket 116 are assembled in a layered fashion to form reaction (or assay) chamber 118 of microposts processing platform 100. Reaction chamber 118 provides a contained microfluidic channel, wherein a defined volume of fluid can be flowed into and out of microposts processing platform 100 (e.g., via fluid ports 120 or directly into gap 114) for subsequent solution-based functionalization reactions.

At a step 225, a first functionalization reaction is performed to add a generic binding agent on the surface of the modified microposts. For example and referring still to FIG. 1A through FIG. 2B, a solution of the first functionalization reagent is flowed into reaction (or assay) chamber 118 (e.g., via fluid ports 120 or directly into gap 114). The first functionalization reagent is, for example, a pegylated amine-reactive biotin reagent, i.e., NHS-PEG-biotin. The N-hydroxysuccinimide ester (NHS) group of the NHS-PEG-biotin reagent reacts with the amine groups on surfaces of the modified microposts 122 to form stable amide bonds. The amphiphilic polyethylene glycol (PEG) spacer arm facilitates solubilization of the biotin-containing molecule in an aqueous solution and minimizes steric hindrance for subsequent biotin binding reactions. Further, the presence of PEG molecules on the surface of microposts 122 substantially improves wetting of reaction (or assay) chamber 118, i.e., PEG molecules on the surface of the PDMS microposts substantially lowers the contact angle between the PDMS microposts and aqueous solutions. The PEG spacer arm also acts as a "bottlebrush" polymer restricting access to the surfaces of microposts 122, thereby reducing non-specific binding to the post surfaces. The biotin functional group at the end of the PEG spacer arm provides a generic binding moiety for the subsequent attachment of a biotinylated-target specific capture agent. The chain length of the PEG spacer is selected to provide sufficient mobility to the terminally bound biotin group. Exemplary molecular weights (MW) for this PEG spacer arm are 500-5000. The first functionalization reaction is performed, for example, using a certain concentration of the first functionalization reagent and reaction conditions to provide a density of PEG-biotin binding sites on the surface of microposts 122 sufficient for subsequent high efficiency capture of a target analyte. In one example, the first functionalization reagent is Biotin-PEG-SCM available from Creative PEGWorks with a molecular weight of 2000.

At a step 230, a blocking (passivation) reaction is performed to block (passivate) unbound amine sites on the biotin-functionalized microposts. For example and referring still to FIG. 1A through FIG. 2B, a solution of the blocking (passivation) reagent is flowed into reaction (or assay) chamber 118 (e.g., via fluid ports 120 or directly into gap 114). The blocking reagent is, for example, an amine-reactive PEG reagent, e.g., NHS-PEG, that reacts with the unbound amine sites and sterically blocks non-specific binding to the surface of microposts 122. The presence of blocking PEG molecules on the surface of microposts 122 also contributes to the improved wetting of reaction (or assay) chamber 118. The chain length of the blocking PEG molecule is selected to be sufficiently shorter than the chain length of the PEG-biotin binding agent (i.e., the first functionalization reagent, NHS-PEG-biotin), with example molecular weights ranging 100-1000. Because the chain length of the blocking PEG molecule is sufficiently shorter than the chain length of the PEG-biotin binding agent, the mobility of the biotin binding group at the end of the PEG arm is maintained. The blocking (passivation) reaction is performed, for example, using a concentration of the blocking reagent and reaction conditions to effectively block (passivate) the surface of microposts 122. In one example, the blocking (passivation) reagent is SS-PEG-SS available from Creative PEGWorks (MW 1000).

At a step 235, a second functionalization reaction is performed to add a binding linker to the biotin-functionalized microposts. For example and referring still to FIG. 1A through FIG. 2B, a solution of the second functionalization reagent is flowed into reaction (or assay) chamber 118 (e.g., via fluid ports 120 or directly into gap 114). The second functionalization reagent is a biotin-binding reagent such as an avidin reagent (e.g., avidin, streptavidin or neutravidin; available from ThermoFisher or Sigma). The biotin-binding reagent (e.g., avidin) binds to the biotin group on the biotin-functionalized microposts 122 and provides a linker for binding a target-specific biotinylated capture agent (e.g., a biotinylated antibody) to microposts 122.

At a step 240, a third functionalization reaction is performed to add a biotinylated target specific capture agent to the PEG-biotin functionalized microposts. For example and referring still to FIG. 1A through FIG. 2B, a solution of the third functionalization reagent is flowed into reaction (or assay) chamber 118 (e.g., via fluid ports 120 or directly into gap 114). The third functionalization reagent is a target-specific biotinylated capture agent such as a biotinylated antibody. The target-specific biotinylated capture agent binds micropost 122 via the biotin-binding linker reagent (e.g., avidin, streptavidin or neutravidin).

At a step 245, the functionalized micro fluidic chamber is dried. In one example, reaction (or assay) chamber 118 is dried using a lyophilization protocol. The use of lyophilization as a technique for drying various reagents (e.g., proteins) is well-known and those of ordinary skill in the art will recognize protocols and/or conditions may be application specific.

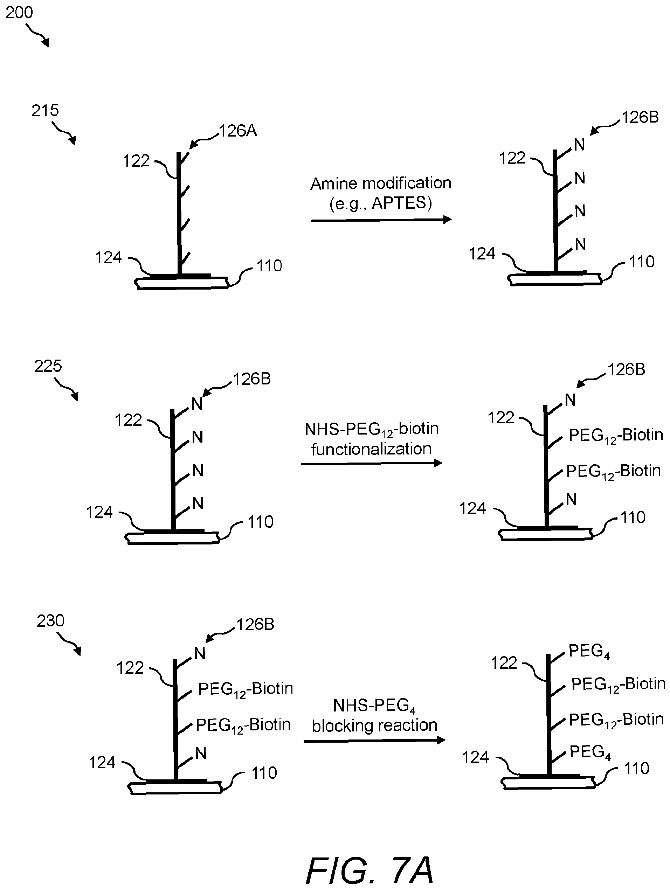

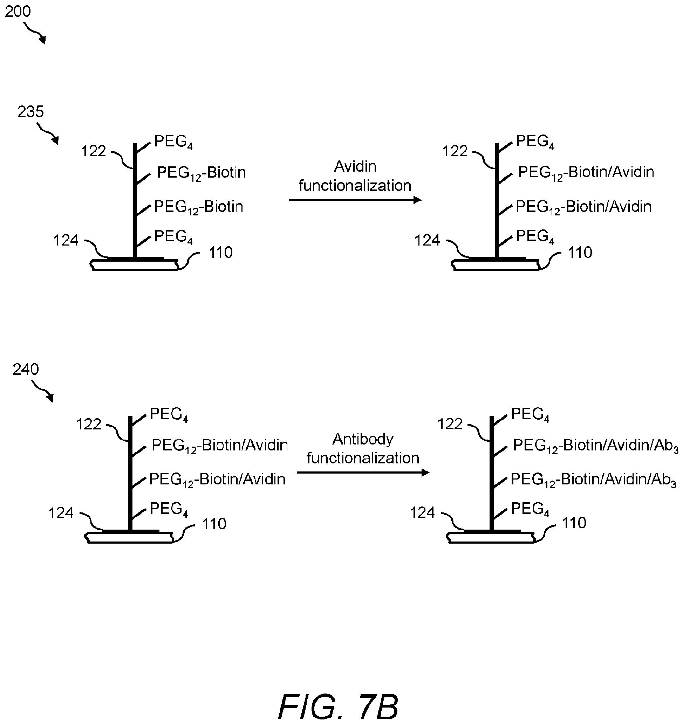

FIG. 7A and FIG. 7B show pictorially some of the steps of method 200 of FIG. 6. In FIG. 7A and FIG. 7B, only a portion of substrate 110 and a single micropost 122 are shown. Namely, at step 210 (not shown), one or more substrates 110 that include surface-attached microposts are provided. For example, two substrates 110 are provided wherein at least one substrate 110 includes an arrangement of surface-attached microposts 122 that are formed, for example, of PDMS.

At step 215, a modification reaction is performed to modify the surface of PDMS micropost 122. For example, the one or more substrates 110 with surface-attached micropost 122 thereon are placed in a reaction chamber (not shown) and exposed to a silanizing agent to convert functional groups 126A (e.g., silanol and radical groups) on the surface of PDMS micropost 122 to amine containing groups 126B. In one example, the silanizing agent is APTES. In one example, the modification reaction is performed using a vapor deposition process, wherein the substrates 110 are placed in a humidity-controlled, sealed chamber and exposed to the silanizing agent (i.e., APTES) at 80°C for from about 1 hour to about 24 hours.

At step 220 (not shown), the two substrates 110 and spacer or gasket 116 are assembled in a layered fashion to form reaction (or assay) chamber 118 of microposts processing platform 100. Reaction chamber 118 provides a contained microfluidic channel, wherein a defined volume of fluid can be flowed into and out of microposts processing platform 100 (e.g., via fluid ports 120 or directly into gap 114) for subsequent solution-based functionalization reactions.

At step 225, the first functionalization reaction is performed to add a generic binding agent on the surface of the amine modified micropost 122. In one example, the first functionalization reagent is an aqueous solution of NHS-PEG-biotin with a molecular weight of 941 or approximately 54 angstroms (i.e., NHS-PEG₁₂-biotin). The NHS group of the NHS-PEG-biotin molecule reacts with the amine group 126B on micropost 122 to covalently bind PEG-biotin to micropost 122. The first functionalization reaction is performed using a concentration of NHS-PEG₁₂-biotin and reaction conditions to provide a certain density of PEG-biotin binding sites on the surface of micropost 122 (i.e., not all amine groups 126B are bound by PEG-biotin). In one example, the first functionalization reaction is performed at room temperature (25°C) for from about 30 minutes to about 1 hour. The biotin functional group at the end of the PEG spacer arm provides a generic binding moiety for the subsequent attachment of a target specific capture agent.

At step 230, the blocking (passivation) reaction is performed to block (passivate) unbound amine groups 126B on PEG-biotin functionalized micropost 122. In one example, the blocking reagent is an aqueous solution of NHS-PEG with a molecular weight of 589 or approximately 29 angstroms (i.e., NHS-PEG₄). In one example, the blocking (passivation) reaction is performed at room temperature (25°C) for from about 30 minutes to about 1 hour. Because the chain length of the blocking PEG molecule is sufficiently shorter than the chain length of the PEG-biotin binding agent, the mobility of the biotin binding group at the end of the PEG arm is maintained.

At step 235, the second functionalization reaction is performed to add a binding linker to the PEG-biotin functionalized micropost 122. In one example, the second functionalization reagent is a biotin-binding reagent such as an avidin reagent (e.g., avidin, streptavidin or neutravidin). The biotin-binding reagent (e.g., avidin) binds to the biotin group on the PEG-biotin functionalized micropost 122. An avidin molecule (e.g., avidin, streptavidin or neutravidin) has four biotin binding sites. When an avidin molecule is bound to a biotin group on micropost 122, three biotin binding sites on the avidin linker molecule remain available for subsequent binding of a target-specific biotinylated capture agent (e.g., 3 biotinylated antibodies, "Abs").

At step 240, the third functionalization reaction is performed to add a biotinylated target specific capture agent to the PEG-biotin/avidin functionalized microposts. The third functionalization reagent is a target-specific biotinylated capture agent such as a biotinylated antibody. The target-specific biotinylated capture agent binds micropost 122 via the biotin-binding linker reagent (e.g., avidin, streptavidin or neutravidin).

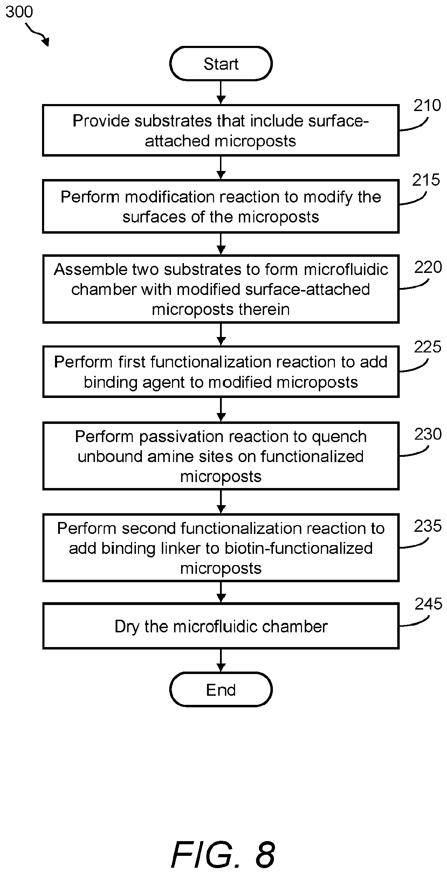

In another embodiment, FIG. 8 illustrates a flow diagram of a method 300, which is another example of the presently disclosed method of surface modification of silicones for specific target binding and high efficiency binding. Method 300 is substantially the same as method 200 shown in FIG. 6 except for the omission of step 240. Namely, method 300 includes the step 210, followed by the step 215, followed by the step 220, followed by the step 225, followed by the step 230, followed by the step 235, and then followed by the step 245; wherein all steps are as described with reference to method 200 of FIG. 6. In this embodiment, microposts processing platform 100 is supplied to an end user with only biotin/avidin functionalized microposts 122. Accordingly, prior to use, the end user would then integrate into their assay protocol a final functionalization step (i.e., as a "run-time" final functionalization reaction) to attach the biotinylated capture agent (e.g., a biotinylated Ab) to microposts 122.

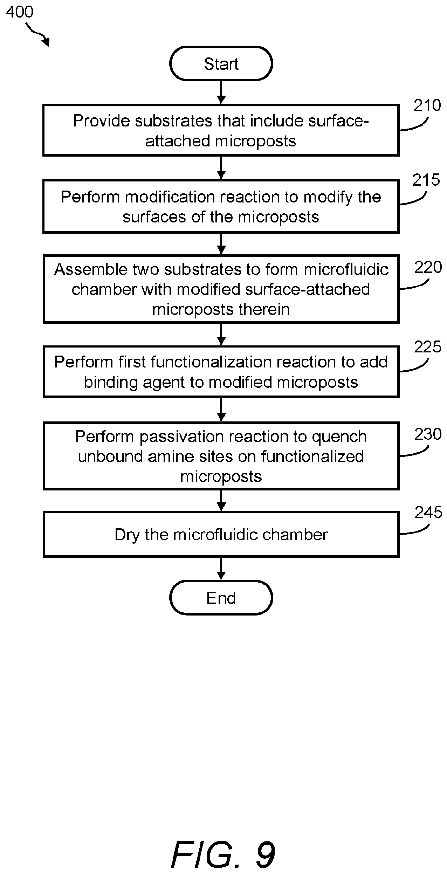

In yet another embodiment, FIG. 9 illustrates a flow diagram of a method 400, which is yet another example of the presently disclosed method of surface modification of silicones for specific target binding and high efficiency binding. Method 400 is substantially the same as method 200 shown in FIG. 6 except for the omission of step 235 and the 240. Namely, method 400 includes the step 210, followed by the step 215, followed by the step 220, followed by the step 225, followed by the step 230, and then followed by the step 245; wherein all steps are as described with reference to method 200 of FIG. 6. In this embodiment, microposts processing platform 100 is supplied to an end user with only biotin functionalized microposts 122. Accordingly, prior to use, the end user would then integrate into their assay protocol two functionalization steps (i.e., as run-time functionalization reactions), a step to add the avidin linker and another step to attach the biotinylated capture agent (e.g., a biotinylated Ab) to microposts 122.

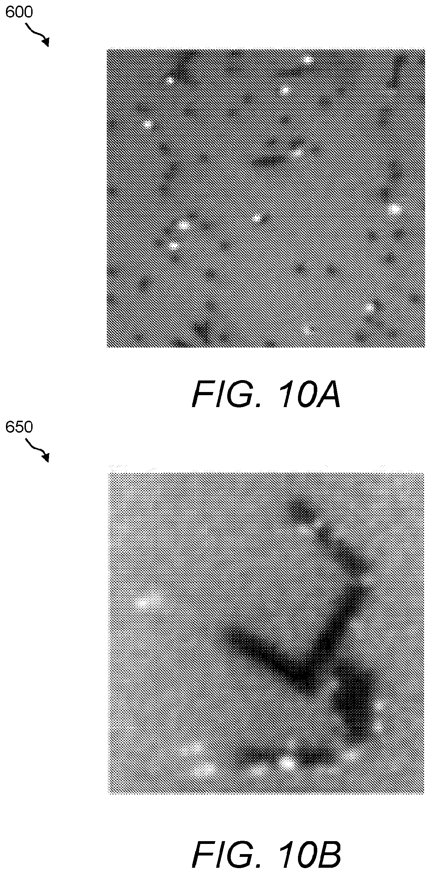

To evaluate the binding chemistry of microposts functionalized with biotinylated capture antibodies (using biotinylated goat anti-mouse IgG) using method 200 of FIG. 6 and microposts processing platform 100 of FIG. 1A through FIG. 2B, analyte capture experiments were performed. For example, FIG. 10A and FIG. 10B are photos of analyte capture experiments performed using beads suspended in a simple buffer matrix and bacteria (i.e., *E. coli*) spiked in whole blood, respectively. Referring now to FIG. 10A, the data demonstrate the capture of beads coated with TNF-α mouse anti-donkey antibody, suspended in a buffer matrix, by microposts functionalized with biotinylated capture antibodies. Referring now to FIG. 10B, the data demonstrate the capture of *E. coli*, spiked in whole blood, by microposts functionalized with biotinylated anti-E.coli O157:H7 capture antibodies (purchased from SeraCare). E.coli were present in the blood at a concentration of approximately 10,000 cells/mL.

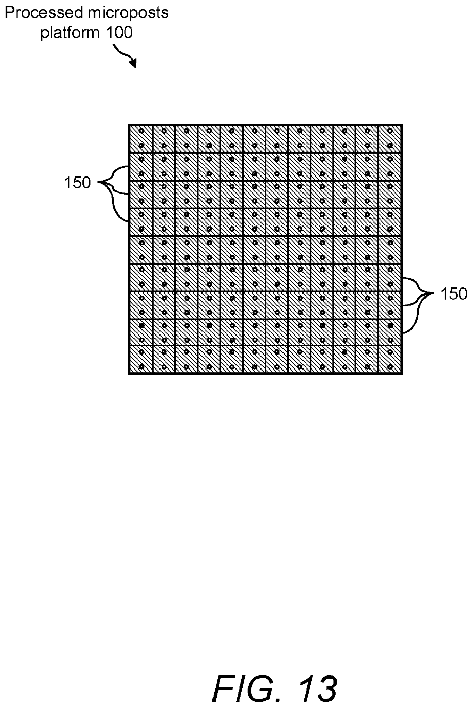

At the completion of any of the presently disclosed micropost surface-functionalization processes (e.g., method 200, method 300, and/or method 400) of surface modification of silicones for specific target binding and high efficiency binding, the microposts processing platform 100 that has been processed can now be called the processed microposts platform 100 that may be delivered to an end user. In one example, FIG. 11 shows a side view of an example of the processed microposts platform 100 in which both substrates 110 include a field or array of functionalized microposts 122. In another example, FIG. 12 shows a side view of an example of the processed microposts platform 100 in which one substrate 110 only includes a field or array of functionalized microposts 122. In both the examples shown in FIG. 11 and FIG. 12, processed microposts platform 100 may be a structure that includes their substrates 110 in their entirety, such as 6-inch or 12-inch diameter substrates that have a substantially continuous field or array of free-standing functionalized microposts 122 across their area. In yet another example, FIG. 13 shows a plan view of an example of the processed microposts platform 100 that is diced into individual microfluidics devices 150, wherein each of the microfluidics devices 150 includes an arrangement of functionalized microposts 122 in a reaction (or assay) chamber. Each of the microfluidics devices 150 can be provided, for example, as a drop-in ready module for easily integrating into any fluidics cartridges or systems of an end user. Again, the state of the functionalized microposts 122 in processed microposts platform 100 and/or microfluidics devices 150 depends on which functionalization method 200, 300, or 400 is used, which may depend on the needs of the end user.

Additionally, while the aforementioned method 200, method 300, and/or method 400 are described as being performed on a substrate-level structure, such as microposts processing platform 100, in other embodiments, the aforementioned method 200, method 300, and/or method 400 can be performed instead on diced devices, such as microfluidics devices 150.

Any portion of the generic binding agent or linking agent may be modified such that it becomes cleavable in order to facilitate the release of the captured object as desired by the user. For example, binding and/or linking agents may be modified so that they are acid, alkyne, hydrazine, sodium dithionite, and photo- cleavable. This is useful for cases where it is important to recover the object that has been captured from the specimen intact. For example, one may wish to capture bacteria from blood by processing a large volume (e.g., 10 mL) in a small (e.g., 10 microliter) chamber, thus concentrating the bacteria. This concentrated specimen may then be released by flushing the small chamber (potentially after washing away remaining blood) with buffer containing a cleaving agent. As a result, one could extract a concentrated (from 10 mL to 10 microliters) and purified (from blood to buffer) specimen.
Embodiments of the present invention are described below as E1 to E75.
E1. A method for functionalizing surface attached microposts to produce a micropost surface for target-specific analyte capture, the method comprising the steps of:
   a. providing a micropost processing platform, wherein the micropost processingplatform comprises microposts that comprise a silicone-based material; and
   b. incorporating one or more functionalizing agents into the silicone-based materialof the microposts;
   whereby the micropost surface for target-specific analyte capture is produced.
E2. The method of E 1, wherein step 1(b) comprises providing a binding system forbinding of a target-specific capture agent, wherein the binding system comprises a generic bindingagent and a linking agent.
E3. The method of E 2, wherein the generic binding agent is biotin and the linking agent is an avidin, and wherein the target-specific capture agent is a biotin-conjugate.
E4. The method of E 2, wherein the avidin is avidin, streptavidin, or neutravidin.
E5. The method of any one of E 3 to E4, wherein the biotin-conjugate is abiotinylated antibody (Ab)).
E6. The method of E 1 or E2, wherein step 1(b) comprises partially or fullyfunctionalizing the surface-attached microposts.
E7. The method of E 6, wherein partially or fully functionalizing the surface- attached microposts comprises anchoring one or more functionalizing agents into the silicone- based material of the microposts.
E8. The method of E 7, wherein the anchoring of the one or more functionalizing agents into the silicone-based material of the microposts comprises grafting, bonding, and/or physio-absorption.
E9. The method of E 6, wherein partially or fully functionalizing the surface- attached microposts comprises providing the one or more functionalizing agents such that the oneor more functionalizing agents sit atop the surface of the microposts.
E10. The method of any one of E 6 to E9, wherein the silicone-based material of themicroposts comprises PDMS.
E11. The method of E 10, wherein the silicone-based material of the microposts comprises one or more amine functional groups, wherein the amine functional group provides ananchor point for subsequent addition of the one or more functionalizing agents.
E12. The method of E 11, wherein the one or more functionalizing agents comprisea binding agent and/or a blocking agent.
E13. The method of E 12, wherein a generic binding agent is added into the silicone-based material of the microposts.
E14. The method of E 13, wherein the generic binding agent comprises a biotin-containing agent.
E15. The method of E 12, wherein the binding agent is a modified lipid thatcomprises a biotin binding group.
E16. The method of E 15, wherein the binding agent is 1,2-dioleoyl-sn-glycero-3-phosphoethanolamine-N-(biotinyl) (Bio Dope).
E17. The method of E 16, wherein the silicone-based material comprises a polymermatrix, and wherein a swelling agent is used to expand the polymer matrix.
E18. The method of E 17, wherein the swelling agent is removed, whereby the functionalizing agent becomes entangled and anchored in the polymer matrix.
E19. The method of any one of E 1 to E18, further comprising the steps of:
   c. reacting functional groups on the surface-attached microposts with a silanizing reagent to form amine-containing groups, whereby amine modified microposts areproduced; and
   d. causing a crosslinking chemical reaction to add the one or more functionalizingagents to the surface of the amine modified microposts.
E20. The method of E 19, wherein the silanizing agent comprises 3- aminopropyltriethoxysilane (APTES) and/or 3-glycidyloxypropyltrimethoxysilane (GOPTS).
E21. The method of E 19 or E20, wherein step (d) comprises the use of N- hydroxysuccinimide ester (NHS) and/or 1-Ethyl-3-(3-Dimethylaminopropyl)carbodiimide (EDAC).
E22. The method of E 19, wherein step (d) comprises the use of click chemistry to conjugate the functionalizing agent to the surface of the microposts.
E23. The method of E 19, wherein step (d) comprises the use of a silanizing agent that comprises a functionalizing agent.
E24. The method of E 23, wherein the silanizing agent that comprises a functionalizing agent comprises silane polyethylene glycol (silane PEG).
E25. The method of E 24, wherein the functionalizing agent comprises a binding group, and further wherein the binding group is a biotin, whereby the functionalizing agent comprises silane polyethylene glycol biotin (silane PEG biotin).
E26. The method of E 19, wherein step (d) comprises the use of a plasma activationprocess to generate reactive groups that can be used to anchor the functionalizing agent to the surface of the microposts.
E27. The method of E 26, the reactive groups comprise hydroxyl groups.
E28. The method of E 19, wherein step (d) comprises passivating the surface of themicroposts, wherein passivating the surface of the microposts comprises reacting unbound aminegroups with a blocking agent.
E29. The method of E 19, wherein step (d) comprises passivating the surface of themicroposts, wherein passivating the surface of the microposts comprises incorporating a blockingagent into the silicone-based material that is used to fabricate the microposts.
E30. The method of E 28 or E29, wherein the blocking agent is selected from the group consisting of: polyethylene glycol (PEG); polyethylene oxide (PEO); a lipid; bovine serum albumin (BSA); polypropylene; and polytetrafluoroethylene (PTFE).
E31. The method of either E 28 or E 29, wherein a first functionalization reactionis performed to add a generic binding agent on the surface of the modified microposts.
E32. The method of E 30, wherein a solution of a first functionalization reagent is flowed into the reaction chamber via fluid ports or directly into the gap.
E33. The method of E 32, wherein the first functionalization reagent is a pegylated amine-reactive biotin reagent (N-hydroxysuccinimide ester (NHS))-PEG-biotin.
E34. The method of E 33, wherein the PEG spacer arm is configured to act as a bottlebrush polymer restricting access to the surfaces of the microposts. whereby non-specific binding to the surfaces of the microposts is reduced.
E35. The method of E 33, wherein the first functionalization reaction is performed using the first functionalization reagent and reaction conditions sufficient to provide a density of PEG-biotin binding sites on the surface of the microposts sufficient for subsequent high efficiency capture of a target analyte.
E36. The method of E 35, the first functionalization reagent is a pegylated amine- reactive biotin reagent, and wherein the reagent reacts with amine groups on surfaces of the modified microposts to form stable amide bonds.
E37. The method of E 36, wherein the chain length of the PEG spacer is selected toprovide sufficient mobility to the terminally bound biotin group.
E38. The method of E 33, wherein the PEG spacer arm is configured to have a molecular weight of between 500 and 5000.
E39. The method of E 33, wherein the chain length of the blocking PEG molecule isselected to be sufficiently shorter than the chain length of the PEG-biotin binding agent to allow for the mobility of the biotin binding group at the end of the PEG arm.
E40. A micropost processing platform comprising microposts that comprise a silicone- based material and configured for performance of the methods of any one of E 1 to E39.
E41. The micropost processing platform of E 40, wherein the micropost processingplatform comprises a microfluidics system comprising:
   i. at least one microfluidic device comprising a reaction chamber, wherein the reaction chamber comprises a micropost field, wherein the micropost field comprises surface-attached magnetically responsive microposts; and
   ii. at least one magnetic-based actuation mechanism provided in close proximity to the magnetically responsive microposts, wherein the at least one magnetic-based actuation mechanism is configured to generate an actuation force sufficient to compel at least some of the magnetically responsive microposts to exhibit motion.
E42. The micropost processing platform of E 40, wherein the reaction chamber comprises at least one contained microfluidic channel, wherein a defined volume of fluid can be flowed into and out of the micropost processing platform via fluid ports for solution-based functionalization reactions.
E43. The micropost processing platform of E 42, wherein the reaction chamber comprises multiple microfluidic channels.
E44. The micropost processing platform of any one of E 40 to E43, wherein the method further comprises reacting functional groups on the surface of the silicone-based materialof the microposts with APTES to form amine-containing groups on amine modified microposts, followed by the use of one or more amine-ester (NHS) chemical reactions to add one or more functional groups to the surface of the amine modified microposts.
E45. The micropost processing platform of E 44, wherein the APTES-modification reaction is performed prior to assembly of the microposts processing platform to form a reaction chamber, and wherein the one or more NHS chemical reactions to add one or more functional groups to the surface of the amine modified microposts are performed in the reaction chamber of the assembled microposts processing platform.
E46. The micropost processing platform of any one of E 40 to E45, comprising two facing substrates of functionalized surface-attached microposts.
E47. The micropost processing platform of any one of E 40 to E45, comprising at least two individual microfluidics devices configured as drop-in ready modules for integration intoa fluidics cartridge or system of an end user.
E48. The micropost processing platform of E 47, wherein the functionalized surface-attached microposts are configured to be provided to the end user at different stages of the functionalization process depending on end-user requirements.
E49. The micropost processing platform of any one of E 40 to E45, comprising a pairof opposing substrates of functionalized surface-attached microposts, wherein the pair of opposing substrates are separated by a gap, thereby forming a reaction chamber between the pair of opposing substrates.
E50. The micropost processing platform of E 49, comprising a spacer or gasket provided between the pair of substrates to form the gap.
E51. The micropost processing platform of E 49, wherein the height of the gap isfrom about 50 µm to about 1 mm.
E52. The micropost processing platform of E 49, configured to allow bulk fluid to be flowed through the reaction chamber by supplying fluid directly to the gap.
E53. The micropost processing platform of E 52, wherein one end of the reactionchamber is an inlet and the other end of the reaction chamber is an outlet.
E54. The micropost processing platform of E 49, comprising fluid ports provided inone or both substrates.
E55. The micropost processing platform of E 54, comprising two fluid portsprovided in one substrate, wherein one fluid port is an inlet and the other inlet port is an outlet.
E56. The micropost processing platform of any one of E 40 to E45, comprising:
   i. at least one microfluidic device comprising a reaction chamber, wherein the reaction chamber comprises a micropost field, wherein the micropost field comprises surface-attached magnetically responsive microposts; and
   ii. at least one magnetic-based actuation mechanism provided in close proximity to the magnetically responsive microposts, wherein the at least one magnetic-based actuation mechanism is configured to generate an actuation force sufficient to compel at least some of the magnetically responsive microposts to exhibit motion.
E57. The micropost processing platform of E 56, wherein each of the at least one microfluidic devices comprises a bottom substrate and a top substrate separated by a gap, therebyforming the reaction chamber between the bottom substrate and the top substrate.
E58. The micropost processing platform of E 57, wherein a spacer or gasket is provided between the bottom substrate and top the substrate to form the gap and define the reactionchamber.
E59. The micropost processing platform of E 56, wherein the top substrate comprisesan inlet port and an outlet port.
E60. The micropost processing platform of E 56, wherein the reaction chamber is sized to hold a desired volume of fluid.
E61. The micropost processing platform of any one of E 56 to E60, wherein the gap has a height of from about 50 µm to about 1 mm.
E62. The micropost processing platform of any one of E 56 to E61, wherein the micropost field is provided on the inner surface of the bottom substrate.
E63. The micropost processing platform of any one of E 56 to E61, wherein the micropost field is provided on the inner surface of the top substrate.
E64. The micropost processing platform of any one of E 56 to E61, wherein the micropost field is provided on both the inner surface of the bottom substrate and the inner surfaceof the top substrate.
E65. The micropost processing platform of any one of E 56 to E64, whereinmicroposts within the reaction chamber are functionalized with target-specific analyte capture elements.
E66. The micropost processing platform of E 65, wherein the microposts are functionalized using a surface-functionalization process.
E67. The micropost processing platform of E 66, wherein surface functionalization of the microposts is performed prior to assembly of the two substrates to form the reaction chamber.
E68. The micropost processing platform of any one of E 45 to E67, comprising opposing microposts, wherein the opposing microposts are configured such that there is no spaceor gap between the tips of the opposing microposts.
E69. The micropost processing platform of E 68, wherein the opposing microposts are interdigitated.
E70. The micropost processing platform of E 69, wherein the interdigitated opposingmicroposts are configured such that the tips of the opposing microposts are set at different planessuch that they overlap.
E71. The micropost processing platform of E 69, wherein the tips of a first set of the opposing microposts are set at a plane p1 and the tips of an opposing set of microposts to the first set of microposts are set at a plane p2.
E72. The micropost processing platform of E 69, wherein the interdigitated opposingmicroposts are configured such that the tips of the opposing microposts are set with substantially no overlap.
E73. The micropost processing platform of E 69, wherein the tips of the opposing microposts are set at the same plane.
E74. The micropost processing platform of E 69, wherein the tips of the opposing microposts are separated by a space or gap.
E75. The micropost processing platform of E 74, wherein the tips of the opposing microposts are configured such that the space or gap between the tips of the opposing micropostsprovides a low resistance flow path through the center of the reaction chamber through which targets can travel and not encounter a micropost.

## Claims

1. A surface for target-specific analyte capture, the surface comprising:
a plurality of microposts extending from the surface, the each of the plurality of microposts comprising,
a silicone-based material functionalized with a binding system for capture of a target specific analyte,
the binding system comprising a blocking agent and a binding agent, wherein the binding agent comprises a linker and a capture agent terminally attached to the linker, wherein the length of the linker is configured to allow movement of the capture agent terminally attached, and wherein the size of the blocking agent is smaller than the size of the linker.

2. The surface of claim 1, wherein the linker is configured to act as a "bottlebrush" polymer restricting access to the surface of each of the plurality of microposts thereby reducing non-specific binding.

3. The surface of claim 1 or 2, wherein:
(i) the linker is a polyethylene glycol (PEG) linker, optionally wherein the PEG linker has a molecular weight from about 500 to about 5000; or
(ii) the linker is avidin or an avidin-conjugate.

4. The surface of claim 1 or 2, wherein the capture agent is biotin or a biotin-conjugate.

5. The surface of claim 1 or 2, wherein the blocking agent is a polyethylene glycol (PEG) blocking agent, optionally wherein the PEG blocking agent has a molecular weight from about 100 to about 1000.

6. The surface of claim 1 or 2, wherein the silicone-based material comprises a polymer matrix.

7. A microfluidic device comprising,
a reaction chamber comprising the surface of claim 1 or 2, wherein the plurality of microposts extending from the surface are magnetically responsive; and
and at least one magnetic-based actuation mechanism in close proximity to the magnetically responsive plurality of microposts, wherein the magnetic-based actuation mechanism is configured to generate an actuation force sufficient to compel at least some of the magnetically responsive plurality of microposts to exhibit motion.

8. The microfluidic device of claim 7, further comprising at least one channel, configured to allow a fluid to flow into and out of the reaction chamber.

9. A method for functionalizing a surface for target-specific analyte capture, the method comprising:
providing a surface comprising a plurality of microposts extending from the surface, wherein the plurality of microposts comprise a silicone-based material;
functionalizing the plurality of microposts by incorporating a binding system for capture of a target specific analyte, wherein incorporating a binding system comprises anchoring to the plurality of microposts a blocking agent and a binding agent, wherein the binding agent comprises a linker and a capture agent terminally attached to the linker, wherein the length of the linker is configured to allow movement of the capture agent terminally attached, and wherein the size of the blocking agent is smaller than the size of the linker.

10. The method of claim 9, wherein anchoring comprises grafting, bonding, and/or physio-absorption.

11. The method of claim 9 or 10, wherein the linker is configured to act as a "bottlebrush" polymer restricting access to the surface of each of the plurality of microposts thereby reducing non-specific binding.

12. The method of claim 9 or 10, wherein:
(i) the linker is a polyethylene glycol (PEG) linker, optionally wherein the PEG linker has a molecular weight from about 500 to about 5000; or
(ii) the linker is avidin or an avidin-conjugate.

13. The method of claim 9 or 10, wherein:
(i) the capture agent is biotin or a biotin-conjugate; or
(ii) the blocking agent is a polyethylene glycol (PEG) blocking agent, optionally wherein the PEG blocking agent has a molecular weight from about 100 to about 1000.

14. The method of claim 9 or 10, wherein the silicone-based material comprises a polymer matrix.

15. The method of claim 9 further comprising reacting a functional group on the plurality of microposts with a silanizing reagent to produce a plurality of amine modified microposts, optionally further comprising adding the one or more functionalizing agents to the amine modified microposts.
